# EUROPEAN PATENT APPLICATION

(11) **EP 4 717 279 A2**
(43) Date of publication of application: **01.04.2026**
(21) Application number: 25201699.3
(22) Date of filing: 11.09.2025
(51) Int. Cl.: A61L 9/03

(54) **DEVICE AND METHOD FOR DISPENSING VOLATILE SUBSTANCES, IN PARTICULAR FRAGRANCES AND/OR ACTIVE SUBSTANCES, AND HEATER**

(30) Priority: 27.09.2024 US 202418899122
(71) Applicant: HAYCO Manufacturing Ltd., Hong Kong (HK)
(72) Inventor: Eschrich, Sven, 73663 Berglen (DE); Shen, Xin, Shenzhen (CN); Zeng, Yiming, Chongqing (CN)
(74) Representative: Liebl, Thomas

(57) **Abstract**

The invention relates to a device (1) for dispensing volatile substances, in particular for dispensing fragrances and/or active ingredients, with at least one container (2) in which a substance to be dispensed is accommodated, the container (2) having a wick (3), which projects out of the container (2) with a free wick end (4) and by means of which the substance to be dispensed is conveyable out of the container (2) into the region of the free wick end (4). The device (1) has an electrical heating device (6) which has a heater with a heater wick recess (8) and which is suitable and designed to be coupled to an electrical power source to heat the heater, wherein the heater wick recess (8) has a heater inlet opening (9) and a heater outlet opening (10), and wherein the free wick end (4) projects into the heater wick recess (8) at least partially in such a way that an air gap running annularly around the free wick end (4) forms a heater air flow channel (11). According to the invention, the heater is formed by a cylindrical heater tube (7) made of a metal or a metal alloy, wherein the outer side (7a) of the cylindrical heater tube (7), facing away from the free wick end (4), and/or the inner side (7b) of the cylindrical heater tube (7), facing the free wick end (4), is at least partially provided with at least one electrically conductive structure (18) that emits heat when energized.

## Description

The invention relates to a device for dispensing volatile substances, in particular fragrances and/or active substances, a method for dispensing volatile substances and a heater.

Such devices are well known in technology and are used in various applications such as room air fresheners, insect repellents or aromatherapy devices. These devices are usually equipped with an electric heating device to vaporize the volatile substances contained in a container and release them into the environment.

Different types of electrical heating devices are known that are used for these purposes. One common type is the Metal Ceramic Heater (MCH), which consists of a metal heating resistor embedded in a ceramic matrix. These heaters are known for their stability and even heat distribution. However, a disadvantage of these systems is their relatively high mass and the limited possibilities for rapid temperature change. WO 2024/114912 A1 describes such a device for vaporizing volatile substances, which uses an elongated, thin-walled heating tube designed as a metal ceramic heater (MCH). This MCH heater encloses the free end of a wick.

Another well-known type is the positive temperature coefficient (PTC) heater, which consists of a ceramic material with a positive temperature coefficient. These PTC elements regulate their temperature independently by increasing their resistance as the temperature rises, thereby reducing heat dissipation. While this self-regulating property is advantageous, PTC heaters are not always optimal due to their lower thermal conductivity and reaction speed, especially when fast and even heating is required.

Finally, metal oxide resistor heaters (e.g. made of aluminium oxide) are also known, which are characterized by their high corrosion resistance and mechanical strength. However, these heaters have the disadvantage of not transferring heat very efficiently.

It is an object of present invention to create a device and a method for dispensing volatile substances, in particular fragrances and/or active substances, which enables an even more effective heat dissipation to a free wick end protruding into a wick recess. A further object of the present invention is to provide a heater for such a device.

This object is solved with the features of the independent claims. Advantageous embodiments are subject of the subclaims referring back thereto.

The invention relates to a device for dispensing volatile substances, in particular for dispensing fragrances and/or active ingredients, having at least one container in which a substance to be dispensed is accommodated, the container having a wick, preferably designed as a capillary element, which projects out of the container with a free wick end and by means of which the substance to be dispensed can be conveyed out of the container into the region of the free wick end.

Furthermore, the device comprises an electrical heating device which has a heater with a heater wick recess and which is suitable and designed to be coupled to an electrical power source for heating the heater, wherein the heater wick recess has a heater inflow opening and a heater outflow opening, and wherein the free wick end projects at least partially into the heater wick recess in such a way that an air gap running annularly around the free wick end forms a heater air flow channel.

According to the invention, it is provided that the heater is formed by a cylindrical heater tube made, preferably entirely, of a metal or a metal alloy, wherein the outer side of the cylindrical heater tube, facing away from the free wick end, and/or the inner side of the cylindrical heater tube, facing the free wick end, is (or are) at least partially provided with at least one electrically conductive structure that emits heat when energized, i.e. when current is flowing through it.

The device according to the invention for dispensing volatile substances is characterized in particular by the use of a cylindrical heater tube made of metal or a metal alloy, which expressly also includes the use of coated metals or metal alloys. The particular advantage of using a metal or metal alloy as the material for the heater tube lies in its high thermal conductivity. Metals enable a rapid temperature rise with comparatively lower energy consumption compared to heaters based on non-metallic materials such as ceramic or plastic. This high thermal conductivity helps to ensure that the heat generated is distributed efficiently and evenly over the entire tube, which optimizes the performance of the device, especially when dispensing fragrances.

Another advantage of metals is their mechanical strength and durability. Metals are more robust and resistant to mechanical stress and wear, which extends the life of the device. In addition, metals offer high formability, which makes it possible to manufacture the tube in various complex geometries to meet specific requirements.

A further particular advantage of the solution according to the invention lies in the fact that the electrically conductive structure applied to the outer side and/or inner side of the cylindrical heater tube ensures uniform, rapid and thus effective heating of the heater tube, so that it radiates at least some of its heat in the direction of the free wick end. If an electrically conductive structure is provided on the inner side of the cylindrical heater tube - either without an electrically conductive structure on the outer side of the cylindrical heater tube or together with an electrically conductive structure on the outer side of the cylindrical heater tube - this can also have the effect that the structure not only heats the cylindrical heater tube itself, but also specifically radiates heat in the direction of the free wick end. This double heating - of both the heater tube and the wick - can be advantageous in certain applications in order to further increase the efficiency of vaporization of the substance to be emitted.

The device according to the invention for dispensing volatile substances is characterized in particular by the use of a cylindrical heater tube made of aluminium.

The particular advantage of using aluminum as the material for the heater tube lies in its high thermal conductivity. Aluminum enables a rapid temperature rise with lower energy consumption compared to heaters based on ceramic or plastic materials. This makes aluminum particularly attractive for applications where efficient heating is required. In principle, other materials with high thermal conductivity could also be considered, such as copper. However, copper is significantly more expensive than aluminum, which represents a considerable cost factor for large production volumes. In addition, the processing of copper is more difficult and costly, especially in the production of thin-walled tubes. Another important point is the risk of short circuits when using copper. Copper does not form a protective oxide layer that has an electrically insulating effect, as is the case with aluminum. Aluminum forms a thin layer of aluminum oxide (Al2O3) on contact with air, which protects the material from corrosion and at the same time provides a degree of electrical insulation. This natural barrier reduces the risk of short circuits when conductors are attached to the surface of the aluminum tube. Copper, on the other hand, requires careful insulation of the conductors to prevent short circuits, which further complicates and increases the cost of the manufacturing process.

Overall, aluminum therefore offers not only economic and thermal advantages, but also practical benefits in terms of avoiding short circuits. This makes aluminum particularly suitable for applications where the integration of conductors or other electrical structures on the surface of the tube is required without the need for additional insulation.

However, the cylindrical heater tube can be manufactured or formed not only from pure respectively largely pure aluminum, whose thermal conductivity at room temperature is generally in the range of approximately 230 to 235 W/mK, but also from an aluminum alloy whose thermal conductivity is at least 200 W/mK at room temperature. The use of an aluminum alloy as the material for the heater tube offers the advantage that alloys can also be used that offer specific mechanical and thermal properties. By choosing a suitable aluminium alloy, the balance between thermal efficiency and mechanical performance can thus be optimized, further increasing the versatility and application possibilities of the device.

The electrically conductive structure can in principle be formed from any suitable material, for example silver, nickel, or conductive pastes, although it is preferable for the electrically conductive structure to be formed from copper, preferably a copper alloy. Copper has the advantage that it has a very high electrical conductivity, which makes it particularly efficient for conducting electricity. Compared to silver, copper is also significantly cheaper and more readily available, making it a practical choice for numerous applications. Although pure copper or coated copper could be used in principle, this is relatively expensive in terms of both material costs and processing. In mass production, the use of copper alloys is therefore more economical. Copper alloys also often offer better mechanical properties and improved corrosion resistance, which further favors their use in durable and robust devices.

According to a further particularly preferred embodiment, it is provided that the electrically conductive structure extends over at least a part of the outer side and/or inner side of the heater tube, preferably extends over the majority of the outer side and/or inner side of the heater tube, most preferably extends over substantially the entire outer side and/or inner side of the heater tube. This makes it possible to ensure that the electrically conductive structure covers the associated surface of the outer side and/or inner side of the cylindrical tube to a desired extent or evenly and, accordingly, to ensure that the heat conduction and radiation via the tube can be optimally adapted to the desired conditions and optimized. This ensures, for example, that the heat is radiated evenly in the direction of the area to be heated.

Furthermore, according to a particularly preferred embodiment, it is provided that the electrically conductive structure is meander-shaped. With such a meander-shaped conductive structure, the effective length of the copper conductor track on the associated surface of the outer side and/or inner side of the cylindrical heater tube can be maximized in a simple and functionally reliable manner. This is particularly advantageous for small diameter heater tubes where the available surface area is limited, making it difficult to accommodate a sufficiently long trace if a simple linear layout were to be used. The meander pattern, which arranges the trace in a repeated, serpentine path, allows the length of the trace to be significantly increased without taking up additional space. This increased length of the track leads directly to a higher electrical resistance. Such a higher electrical resistance is particularly desirable in this case to control the current flow. Resistance is a fundamental property of an electrically conductive structure respectively of an electrical conductor that determines how much current flows through the conductor at a given voltage. A low resistance would mean that a very high current flows at the same voltage. In practical applications, however, a high current flow can be problematic:

Especially when using Li-ion batteries, which are widely used in many modern devices due to their high energy density and rechargeability, excessive power consumption can put a lot of strain on the battery. This can shorten the life of the battery, lead to overheating and, in the worst case, damage the electronics. Excessive current flow can also overheat the conductive tracks, especially if they are made of a copper material, which can lead to "burn out". This means that the conductor track becomes so hot that it is damaged or completely destroyed, which can lead to a malfunction of the entire device. This risk is particularly high if the conductor track is arranged in a small and limited space, as in the case of the cylindrical heater tube, where heat dissipation is restricted.

In order to avoid these problems, it is therefore generally desirable in the present case to provide a target resistance of at least 0.3 to 0.4 ohms with the electrically conductive structure - irrespective of its specific design. Such a resistance range is generally suitable for ensuring a moderate current flow that does not overload the power supply system and at the same time minimizes the risk of burning through the conductor track.

In principle, an embodiment of the electrically conductive structure in a meander shape also allows for a particularly simple formation of contact points, preferably solder points, along a tube edge. These contact points, preferably solder points, can for example be arranged adjacent to each other on one edge of the or at tube edge regions that are spaced apart from each other.

Furthermore, an optional embodiment is advantageous wherein the meander-shaped electrically conductive structure is formed by an arrangement of parallel conductor tracks, with the ends of each conductor track being alternately connected to the end of the immediately preceding and the immediately following conductor track, and wherein it is preferably provided that each conductor track itself has a wave form or a meander form, preferably a meander form with a plurality of U-shaped meander segments aligned up in the longitudinal direction of the conductor track, which are formed by horizontal and vertical conductor sections. These structures each maximize the conductor path length in a limited space and are designed in such a way that they enable uniform heating of the cylindrical heater tube when current flows.

It is also understood that in the event that the electrically conductive structure is arranged both on the surface of the inner side and on the surface of the outer side, a corresponding connection (also referred to as a "via") must be provided to ensure the electrical connection.

Furthermore, according to an optional embodiment, the electrically conductive structure is only provided on the outer side of the cylindrical heater tube. Placing the electrically conductive structure on the outer side of the tube is easier to manufacture than placing it on the inner side. The outer surface of the tube is easily accessible for production processes such as applying conductive tracks, coating or attaching connections. This reduces complexity and lowers production costs. Another advantage is that placing the conductive track on the outer side of the tube avoids the risk of contamination with the substance to be dispensed.

A particularly preferred embodiment is one in which the heater tube is formed by an elongated and/or thin-walled cylindrical shell. A significant advantage of such elongated and/or thin-walled cylindrical heater tube is that it can be heated to a desired temperature very quickly, which results in a very fast response of the device as a whole. This has a very beneficial effect on the performance of the device as a whole. Thin-walled means here a tube which, viewed in the longitudinal direction of the tube, has a large extension, while the tube wall forming the tube has, in contrast, a significantly smaller extension or wall thickness. Accordingly, the wall thickness of the tube is small or very small compared to the length of the tube viewed in the longitudinal direction of the tube. Accordingly, for forming the elongated and/or thin-walled heater tube, it has proven to be particularly advantageous if the ratio between the wall thickness of the heater tube and its length is at least 1:5, preferably 1:5 to 1:100, most preferably 1:10 to 1:50. With these values, the above advantages can be achieved in a particularly simple and functionally reliable manner.

This applies analogously to a further particularly preferred embodiment, according to which it is provided that the cylindrical heater tube has a wall thickness, preferably a wall thickness which is constant in the circumferential direction, of 0.2mm to 5.0mm, preferably of 0.3mm to 3.0mm, most preferably of 0.3mm to 2.0mm.

And further this applies analogously also to a particularly preferred embodiment, according to which it is provided that the cylindrical heater tube has a length of from 5.0mm to 50.0mm, preferably from 10.0mm to 40.0mm, most preferably from 15.0mm to 35.0mm.

The cylindrical heater tube can in principle have any suitable or desired cross-sectional geometry, i.e. for example also an angular, in particular polygonal, cross-sectional geometry, which may also depend on the external geometry of the free wick end. However, a particularly preferred embodiment - in particular also for reasons of production technology and/or for reasons of a conventional, circular-cylindrical outer geometry of the free wick end - is one in which the heater tube has a circular-cylindrical outer circumference and/or a circular-cylindrical inner circumference. And in this context, an embodiment having a circular cylindrical outer circumference and a circular cylindrical inner circumference with the same wall thickness throughout in the circumferential direction is preferred. Such a shape of the heater tube can be produced in a particularly simple and functionally reliable manner.

For simple and functionally reliable manufacture, which can also be carried out inexpensively, it is also particularly advantageous if the heater tube has an identical cross-section on the inner side and/or on the outer side which is continuous in the longitudinal direction. Particularly in the event that flow velocities or special inflow conditions may have to be taken into account, however, it is also possible for the heater tube to have areas with different cross sections on the inner side and/or on the outer side, relative to the longitudinal direction.

The air gap surrounding the free wick end plays an important role in substance delivery via the free wick end, which projects into the heater tube. The inventors found out that, depending on the specific application, very good substance delivery to the air flow in the air gap results if the air gap surrounding the free wick end has at least a gap width of 0.5mm to 5.0mm at any point, preferably of 1.0mm to 4.0mm, most preferably of 2.0mm to 3.0mm at any point.

As shown in the previous explanations, there are various possibilities and options for the geometric design of the heater tube. This applies analogously to the wick or the free wick end, which preferably has a circular cylindrical outer circumference. And in this context, it is then particularly advantageous if the heater tube has a circular-cylindrical inner circumference, preferably with the free wick end being accommodated approximately centrally and/or centred in the heater tube and thus preferably the air gap surrounding the free wick end in an annular or ring-shaped manner with a substantially equal or uniform gap width. In addition to excellent manufacturability, such a specific design also results in optimized air flow in the air gap with optimized substance delivery to the air flow flowing in the air gap.

According to another particularly preferred embodiment, the cylindrical heater tube is provided with a thermal insulation on its outer side, preferably in such way that the outer side of the cylindrical heater tube is sheathed with a thermal insulation. Advantageously, this allows the heat to be directed specifically inwards towards the wick or the free wick end and any undesired heat emission to the environment can be prevented.

In principle, the thermal insulation can be formed in different ways. Thus, according to a first embodiment, it can be provided that the thermal insulation is formed by a thermally insulating coating which is applied to the outer side of the cylindrical heater tube, possibly covering any electrically conductive structure present there. A thermally insulating coating is a particularly space-saving solution, as it can be applied directly to the surface of the heater tube, so that no additional space is required for separate insulating elements. This embodiment also allows uniform coverage and adaptation to the specific geometry of the heater tube, which is particularly advantageous for complex shapes. And, if the coating covers any electrically conductive structure on the outer side, it provides additional protection against mechanical damage and environmental influences without impairing the electrical function.

According to an additional or alternative solution, the thermal insulation can also be formed by using a separate thermal insulating element. This technical solution increases design flexibility and can also be integrated directly into a housing. Additional coating measures can be advantageously dispensed with. For example, the thermal insulating element may be formed by a housing of the device, preferably by a holding device for the cylindrical heater tube forming part of the housing, or the thermal insulating element may be formed by a separate component arranged in the interior of a housing of the device. On the one hand, this solution makes optimum use of the available space by integrating the insulation as part of the housing or the holding device. On the other hand, a separate component can be easily exchanged and replaced, for example if this is desired in conjunction with an active influence on heat dissipation.

The thermal insulating element forming an outer circumferential sheathing can also sheathe the outer side of the heater tube in a contact joint or have a defined gap between it and the outer side of the heater tube.

Accordingly, according to a particularly advantageous specific embodiment, the device has a housing which has at least one housing outlet opening for the air flow enriched with the substance to be dispensed, wherein the heater tube is held and/or supported in the housing. The container can be part of the housing or be designed as a separate component that is connected to the housing, in particular detachably connected. According to a further optional embodiment, it is particularly advantageous if the housing specifically has a holding device which holds and/or supports the heater tube in the area of the heater inlet opening and/or in the area of the heater outlet opening, preferably embracing it at least in certain circumferential areas.

In order to ensure that the air flowing off via the heater air flow duct and enriched with substance can reach the environment outer side the housing in a functionally safe and unhindered manner, it is preferably provided that the heater outflow opening of the heater air flow duct is arranged adjacent, preferably directly adjacent, to the at least one housing outlet opening.

Furthermore, according to a particularly preferred optional design, the heater outflow opening is adjoined by an outlet nozzle which tapers conically in the direction of the heater outflow opening and has an outlet nozzle opening. This allows the air flow enriched with substance to be directed towards the housing outlet opening in a particularly simple and advantageous way. In addition, the tapered nozzle increases the speed of the outflowing air flow, which further increases the efficiency of the substance delivery.

It is particularly preferable for the outlet nozzle to be supported and mounted on the heater tube and/or in the housing. The support and mounting of the outlet nozzle on the heater tube and/or in the housing ensures a simple, stable and precise arrangement and alignment of the nozzle.

In principle, the outlet nozzle can be made of any suitable material. However, it is particularly preferable that it is made of the same material as the cylindrical heater tube, i.e. aluminum or an aluminum alloy with a high thermal conductivity of at least 200 W/mK. This ensures even heat distribution, avoids thermal bridges and simplifies production, as both components require similar processing and manufacturing techniques.

According to a further particularly preferred embodiment, the housing has an air guiding device by means of which an air flow can be guided in the direction of the heater inlet opening, in particular in such a way that an essentially annular air flow flows into the heater air flow channel. With such an air guiding device, a particularly advantageous air guiding and control of the air flow is therefore achieved in the manner required for a particularly effective air inflow into the heater air flow channel.

The air flow is preferably generated by an air flow generating device by means of which air, in particular ambient air, can be drawn or sucked in and the air flow can be generated. At this point, it should be expressly mentioned and clarified that the term "air" in the context of the present invention is to be expressly understood in a broad and comprehensive sense and is also intended to include gases other than ambient air, for example gases which can be delivered via a separate gas cartridge, although it is preferred that ambient air is drawn or sucked in by means of the air flow generating device and thus an ambient air flow is generated which flows in the direction of the heater tube. The air flow generating device is preferably formed by at least one fan. Such a fan is eminently suitable for generating the desired air flow. Furthermore, such a fan is functionally reliable in operation and inexpensive to purchase and manufacture.

According to a particularly preferred specific embodiment, it is provided that the housing-side air guiding duct is coupled to the at least one air flow generating device, preferably to the at least one fan, most preferably a high speed fan, via which air can be drawn in and delivered, preferably as a high speed air flow, to the housing-side air guiding duct. This results in an overall compact design of the device, wherein it is further particularly preferred if the air flow generating device is accommodated in the housing and air is drawn in from outer side the housing.

In addition, according to a particularly preferred, high-quality device, the heating device can be coupled to a control device by means of which the heating device can be activated and/or deactivated in a controlled manner to heat the heater tube. This results in an overall increased flexibility in the use of the device, which can be operated individually by an operator.

The problem according to the invention is further solved by a method for operating a device for dispensing volatile substances, in which at least one container is provided in which a substance to be dispensed is accommodated, the container having a wick, preferably designed as a capillary element, which projects out of the container with a free wick end and by means of which the substance to be dispensed is conveyed out of the container into the region of the free wick end. Furthermore, an electrical heating device is provided, which has a heater with a heater wick recess and which is coupled to an electrical power source for heating the heater, wherein the heater wick recess has a heater inlet opening and a heater outlet opening. The free wick end protrudes at least partially into the heater wick recess in such a way that an annular air gap running around the free wick end forms a heater air flow channel, along which an air stream flowing in via the heater inlet opening flows to the heater outlet opening, enriched with the substance emitted by the free wick end, and flows out there as an air stream enriched with substance. According to the invention, the heater is formed by a cylindrical heater tube made, preferably entirely, of a metal or a metal alloy, wherein the outer side of the cylindrical heater tube, facing away from the free wick end, and/or the inner side of the cylindrical heater tube, facing the free wick end, is (or are) at least partially provided with at least one electrically conductive structure that emits heat when energized (i.e. in the current-carrying state), thereby heating the cylindrical heater tube, so that the cylindrical heater tube radiates at least some of its heat in the direction of the free wick end.

The advantages resulting from the method according to the invention correspond identically to those of the device, so that in this respect reference is made to the explanations given above.

Finally, a heater for a device for dispensing volatile substances is also claimed, in particular for a device for dispensing volatile substances as described above. This heater has a heater wick recess which has a heater inlet opening and a heater outlet opening which is suitable and designed to receive a free wick end of a wick (preferably designed as a capillary element), which can be part of a container in which a substance to be dispensed is accommodated, and by means of which the substance to be dispensed can be conveyed from the container into the region of the free wick end. According to the invention, it is provided that the heater is formed by a cylindrical heater tube made, preferably entirely, of a metal or a metal alloy, wherein the outer side of the cylindrical heater tube and/or the inner side of the cylindrical heater tube is (or are) at least partially provided with at least one electrically conductive structure that emits heat when energized, i.e. in the current-carrying state when current is flowing through it.

The advantages resulting from the heater according to the invention correspond identically to those of the device and the method, so that in this respect reference is made to the explanations given above.

The invention is explained in more detail below by way of example only, with reference to drawings.

It shows:
- Fig. 1: a schematic sectional drawing of an exemplary embodiment of a device for dispensing volatile substances according to the invention,
- Fig. 2: a enlarged view of a part of the sectional drawing of Fig. 1,
- Fig. 3: an enlarged detailed and perspective view of an exemplary embodiment of a cylindrical heater tube,
- Fig. 4: a diagram showing a comparison between MCH heater (solid line) and a heater tube according to the present invention.

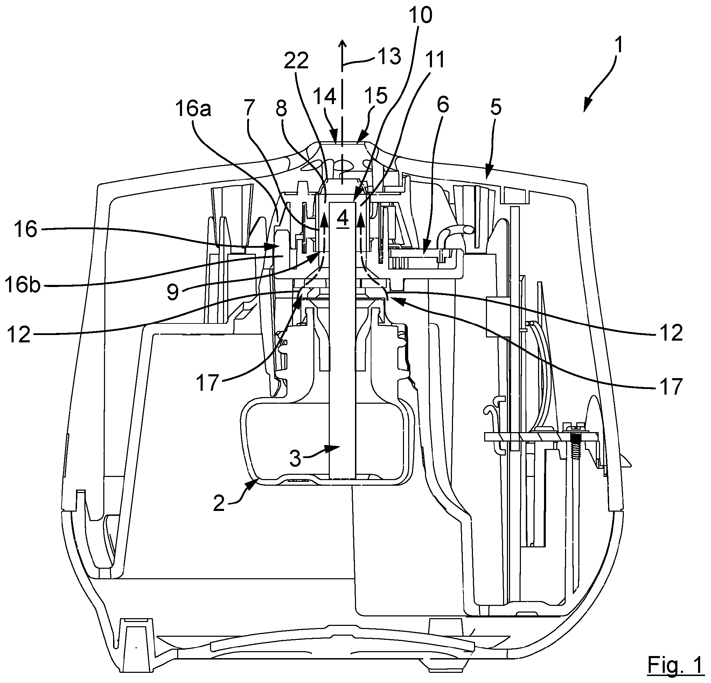

Figure 1 shows a schematic sectional drawing of an exemplary embodiment of a device 1 according to the invention for dispensing volatile substances, such as fragrances and/or active substances, which has a container 2 in which a substance to be dispensed, which is not shown in more detail here, is accommodated.

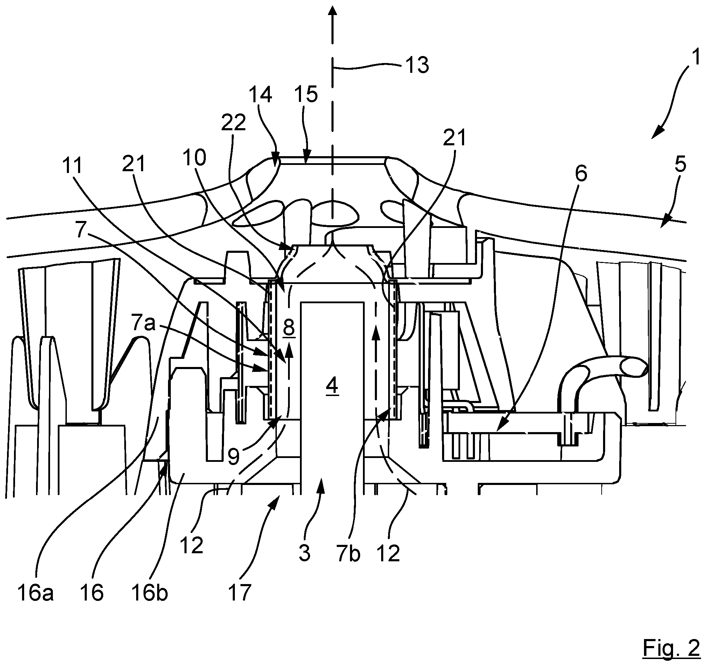

As can be seen in particular from Figure 2, which shows an enlarged detailed view of the upper region of the device 1, the container 2 has a wick 3, which is designed here as a capillary element and protrudes from the container 2 with a free wick end 4. By means of this wick 3, the substance to be dispensed can be conveyed by capillary action from the container 2 into the region of the free wick end 4.

The device 1 further comprises a housing 5 to which the container 2 can be detachably connected, as exemplified here.

As can be further seen from Figures 1 and 2, the device 1 further comprises a heating device 6 which in turn comprises a heater formed by an elongated, thin-walled and cylindrical heater tube 7.

The heater tube 7 has a wick recess 8 as well as a heater inflow opening 9 and a heater outflow opening 10, the free wick end 4 projecting into the wick recess 8 at least partially in such a way that an air gap running ring-shaped around the free wick end 4 forms a heater air flow channel, along which an air flow 12 flowing in via the heater inflow opening 9 flows to the heater outflow opening 10 while being enriched with the substance emitted by the free wick end 4 and flows out there as an air flow 13 enriched with substance.

In the example shown here, the ratio between the wall thickness of the heater tube 7 and its length, for forming the elongated and thin-walled heater tube 7, is approximately 1:50, i.e., the heater tube 7 here has only an exemplary wall thickness of approximately 0.6 mm and a length of approximately 30.0 mm. Of course, other dimensions for forming the elongated, thin-walled and cylindrical heater tube 7 are also possible here, as has also been explained in detail previously in the general part of the description.

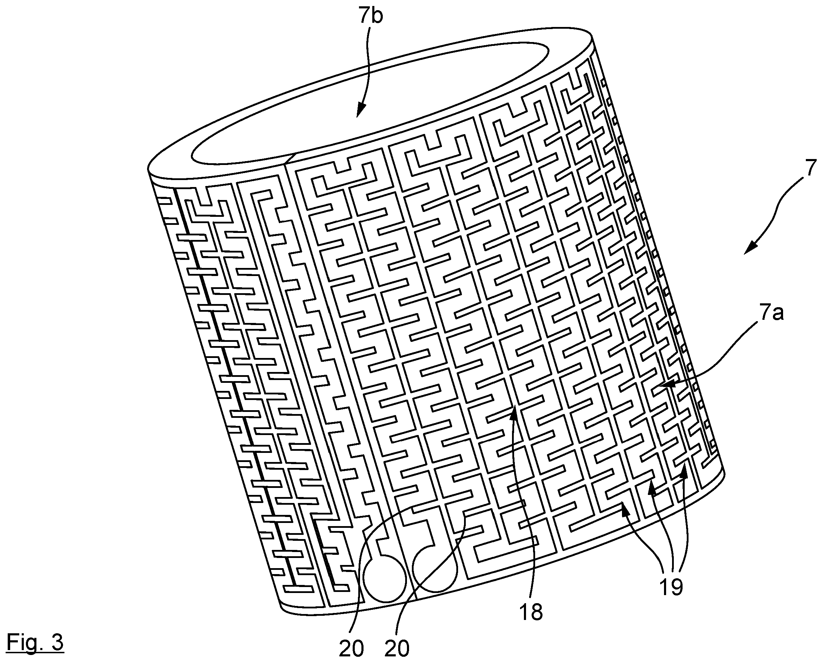

As can be best seen from Figure 3, showing an enlarged detailed and perspective view of an exemplary embodiment of a cylindrical heater tube 7, the heater tube 7 here has both a circular-cylindrical outer circumference and a circular-cylindrical inner circumference, with the same wall thickness throughout in the circumferential direction. In addition, the heater tube 7 has a longitudinally continuous, identical cross-section on both the inner side and the outer side. Here, too, other geometries are of course again possible in principle, although the variant shown in the exemplary embodiment is characterized by a particularly advantageous and simple manufacture.

The free wick end 4 also has here - again only exemplarily - a circular-cylindrical outer circumference and is received approximately centrally respectively centred in the heater tube 7, so that the air gap forming the heater air flow channel 11 surrounds the free wick end 4 ring-shaped as well as with a substantially equal gap width.

The cylindrical heater tube 7 is preferably made entirely of aluminium. Alternatively, the cylindrical heater tube 7 can also be made entirely of an aluminium alloy that has a thermal conductivity of at least 200 W/mK. In the exemplary embodiment shown in Figure 3, only the outer side 7a of the cylindrical heater tube 7, facing away from the free wick end 4, is provided with an electrically conductive structure 18 that emits heat when energized. In the example shown in Figure 3, the electrically conductive structure 18 is formed from a copper alloy that has a resistance of at least 0.3 to 0.4 ohms. The electrically conductive structure 18 is meander-shaped and extends in the exemplary embodiment of Figure 3 over substantially the entire outer side 7a of the heater tube 7. Alternatively, or in addition, the inner side 7b of the cylindrical heater tube 7, facing the free wick end 4, could also be provided with an electrically conductive structure, but this is not shown here.

The meander-shaped electrically conductive structure 18 is formed by an arrangement of parallel conductor tracks 19, where the ends of each conductor track are alternately connected to the end of the immediately preceding and the immediately following conductor track 19. Each conductor track 19 itself has a wave form or a meander form, preferably as shown in the exemplary embodiment of Figure 3, a meander form with a plurality of U-shaped meander segments 20 aligned in the longitudinal direction of the respective conductor track 19, which meander segments 20 are formed by horizontal and vertical conductor sections.

As shown schematically with dotted lines in Figure 2, the cylindrical heater tube 7 may be provided with thermal insulation 21 on its outer side 7a. The thermal insulation 21 here is formed by a thermally insulating coating that is applied to the outer side 7a of the cylindrical heater tube 7, also covering the electrically conductive structure 18.

As can be best seen from Figure 1, the housing 5 has a housing outlet opening 14 for the air flow 13 enriched with the substance to be discharged, which is arranged directly adjacent to the heater outflow opening 10.

As can be seen from Figures 1 and 2, the heater outflow opening 10 is adjoined by an outlet nozzle 22 which tapers conically in the direction of the heater outflow opening 10 and has an outlet nozzle opening. Here, the outlet nozzle 22 is by way of example supported and mounted on the heater tube 7 and in the housing 5. In principle, the outlet nozzle 22 can be made of any suitable material. However, it is particularly preferable that it is made of the same material as the cylindrical heater tube, i.e. aluminum or an aluminum alloy with a high thermal conductivity of at least 200 W/mK.

As can be seen in particular from Figures 1 and 2, the housing 5 further has a holding device 16 which circumferentially grips, holds and supports the heater tube 7.

Furthermore, the holding device 16 is formed of multiple parts, here exemplarily of two parts, namely of an upper holding part 16a and a lower holding part 16b. Between the upper holding part 16a and the lower holding part 16b of the holding device 16 there is a horizontal separating plane which allows easy assembly of the holding device 16 together with the heater tube 7, which is inserted into a central insertion recess of the holding device 16, which also forms the wick recess 8.

Furthermore, the device 1 has an air guiding device 17, which is part of an air guiding duct on the housing side, via which the air flow 12 flows in the direction of the heater inflow opening 9.

To generate the air flow 12, an air flow generating device (not shown) in form of a fan might be provided.

The heating device 6 can also be coupled to a control device (not shown) by means of which the heating device 6 can be activated or deactivated in a controlled manner.

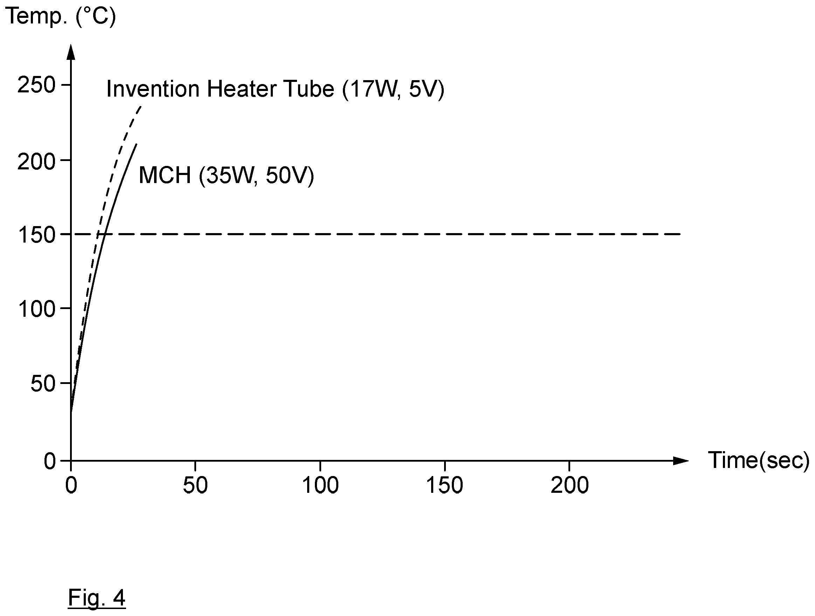

Fig. 4 is a diagram showing a comparison between a Metal Ceramic Heater (35 Watts, 50V) of the state of the art, represented by a solid line, and a heater tube 7 (17 Watts, 5V) according to the present invention, represented by a dotted line. The diagram illustrates the differences in thermal conductivity and efficiency of heat generation between the two systems, showing that with a heater tube according to the present invention, a similar or even faster temperature rise can be achieved with less power.

A dispensing technology as described in the context of the present invention can in principle be used and applied anywhere, but it is particularly preferred to be used in conjunction with air care or pest control products, like for example with perfume or mosquito repellent products.

### List of reference signs

- 1: device
- 2: container
- 3: wick
- 4: free wick end
- 5: housing
- 6: heating device
- 7: heater tube
- 7a: outer side
- 7b: inner side
- 8: wick recess
- 9: heater inflow opening
- 10: heater outflow opening
- 11: heater air flow channel
- 12: air flow
- 13: airflow enriched with substance
- 14: housing outlet opening
- 15: housing outflow channel
- 16: holding device
- 16a: upper holding part
- 16b: lower holding part
- 17: air guiding device
- 18: electrically conductive structure
- 19: conductor track
- 20: meander segment
- 21: thermal insulation
- 22: outlet nozzle

## Claims

1. Device (1) for dispensing volatile substances, in particular for dispensing fragrances and/or active ingredients,
with at least one container (2) in which a substance to be dispensed is accommodated, the container (2) having a wick (3), which projects out of the container (2) with a free wick end (4) and by means of which the substance to be dispensed is conveyable out of the container (2) into the region of the free wick end (4),
with an electrical heating device (6) which has a heater with a heater wick recess (8) and which is suitable and designed to be coupled to an electrical power source to heat the heater,
wherein the heater wick recess (8) has a heater inlet opening (9) and a heater outlet opening (10),
wherein the free wick end (4) projects into the heater wick recess (8) at least partially in such a way that an air gap running annularly around the free wick end (4) forms a heater air flow channel (11),
**characterized in that**
the heater is formed by a cylindrical heater tube (7) made of a metal or a metal alloy, wherein the outer side (7a) of the cylindrical heater tube (7), facing away from the free wick end (4), and/or the inner side (7b) of the cylindrical heater tube (7), facing the free wick end (4), is at least partially provided with at least one electrically conductive structure (18) that emits heat when energized.

2. Device according to claim 1, **characterized in that** the metal is formed by aluminium.

3. Device according to claim 1, **characterized in that** the metal alloy is formed by an aluminium alloy which has a thermal conductivity of at least 200 W/mK.

4. Device according to any one of the preceding claims, **characterized in that** the electrically conductive structure (18) is formed from copper, preferably from a copper alloy.

5. Device according to any one of the preceding claims, **characterized in that** the electrically conductive structure (18) has a resistance of at least 0.3 to 0.4 ohms.

6. Device according to any one of the preceding claims, **characterized in that** the electrically conductive structure (18) extends over at least a part of the outer side (7a) and/or inner side (7b) of the heater tube (7), preferably extends over the major part of the outer side (7a) and/or inner side (7b) of the heater tube (7), most preferably extends over substantially the entire outer side (7a) and/or inner side (7b) of the heater tube (7).

7. Device according to any one of the preceding claims, **characterized in that** the electrically conductive structure (18) is meander-shaped.

8. Device according to claim 7, **characterized in that** the meander-shaped electrically conductive structure (18) is formed by an arrangement of parallel conductor tracks (19), where the ends of each conductor track (19) are alternately connected to the end of the immediately preceding and the immediately following conductor track (19).

9. Device according to any one of the preceding claims, **characterized in that** the electrically conductive structure (18) is provided only on the outer side (7a) of the cylindrical heater tube (7).

10. Device according to any one of the preceding claims, **characterized in that** the heater tube (7) is formed by an elongated and/or thin-walled cylindrical shell.

11. Device according to any one of the preceding claims, **characterized in that** the cylindrical heater tube (7) is provided on its outer side (7a) with a thermal insulation (21), preferably in such way that the outer side (7a) of the cylindrical heater tube (7) is sheathed with a thermal insulation (21).

12. Device according to any one of the preceding claims, **characterized in that**
- a housing (5) is provided which has at least one housing outlet opening (14) for the air flow (13) enriched with the substance to be dispensed,
- the heater tube (7) is supported and/or mounted in the housing (5), and
- the container (2) is part of the housing (5) or is designed as a separate component which is connected to the housing (5), in particular is detachably connected.

13. Device according to any one of the preceding claims, **characterized in that** the heater outflow opening (10) is adjoined by an outlet nozzle (22) which tapers conically in the direction of the heater outflow opening (10) and has an outlet nozzle opening.

14. Device according to claim 12 or 13, **characterized in that** the housing (5) has an air guiding device (17) by means of which an air flow is guidable in the direction of the heater inlet opening (9), preferably in such a way that a substantially annular air flow flows into the heater air flow channel (11).

15. Method for operating a device (1) for dispensing volatile substances, in particular method for operating a device (1) according to any one of the preceding claims,
with at least one container (2) in which a substance to be dispensed is accommodated, the container (2) having a wick (3), which projects out of the container (2) with a free wick end (4) and by means of which the substance to be dispensed is conveyed out of the container (2) into the region of the free wick end (4),
with an electrical heating device (6), which has a heater with a heater wick recess (8) and which is coupled to an electrical power source for heating the heater,
wherein the heater wick recess (8) has a heater inlet opening (9) and a heater outlet opening (10),
wherein the free wick end (4) projects at least in certain areas into the heater wick recess (8) in such a way that an air gap running annularly around the free wick end (4) forms a heater air flow channel (11), along which an air flow (12) flowing in via the heater inlet opening (9) flows, enriched with the substance emitted by the free wick end (4), to the heater outlet opening (10) and flows out there as an air flow (13) enriched with substance,
**characterized in that**
the heater is formed by a cylindrical heater tube (7) made of a metal or a metal alloy, wherein the outer side (7a) of the cylindrical heater tube (7), facing away from the free wick end (4), and/or the inner side (7b) of the cylindrical heater tube (7), facing the free wick end (4), is at least partially provided with at least one electrically conductive structure (18) that emits heat when energized, thereby heating the cylindrical heater tube (7), so that the heater tube (7) radiates at least some of its heat in the direction of the free wick end (4).

16. A heater for a device for dispensing volatile substances, in particular for use in a device (1) for dispensing volatile substances according to any one of claims 1 to 14,
with a heater wick recess (8), which has a heater inflow opening (9) and a heater outflow opening (10), which is suitable and designed to receive a free wick end (4) of a wick (3), which is part of a container (2) in which a substance to be dispensed is accomodated, and by means of which the substance to be dispensed is conveyable from the container (2) into the region of the free wick end (4),
**characterized in that**
the heater is formed by a cylindrical heater tube (7) made of a metal or a metal alloy, wherein the outer side (7a) of the cylindrical heater tube (7) and/or the inner side (7b) of the cylindrical heater tube (7) is at least partially provided with at least one electrically conductive structure (18) that emits heat when energized.
